# EUROPEAN PATENT SPECIFICATION

(11) **EP 3 075 249 B1**
(45) Date of publication and mention of the grant of the patent: **22.07.2020**
(21) Application number: 16000729.0
(22) Date of filing: 30.03.2016
(51) Int. Cl.: A01P 3/00, C12R 1/465, C12N 11/00, C12N 1/00, A01N 63/28

(54) **PRODUCT FOR CONTROLLING PHYTOPATHOGENIC FUNGI THAT CAUSE GRAPEVINE WOOD DISEASES AND METHOD FOR THE APPLICATION THEREOF IN GRAPEVINE GRAFTS**
PRODUKT ZUR BEKÄMPFUNG VON PHYTOPATHOGENEN, REBENHOLZKRANKHEITEN VERURSACHENDEN PILZEN UND VERFAHREN ZUR ANWENDUNG DAVON IN REBENTRANSPLANTATEN
PRODUIT POUR LUTTER CONTRE DES CHAMPIGNONS PHYTOPATHOGÈNES RESPONSABLES DE MALADIES DE LA VIGNE ET PROCÉDÉ POUR SON APPLICATION DANS DES GREFFES DE VIGNE

(30) Priority: 01.04.2015 ES 201530448
(43) Date of publication of application: 05.10.2016
(73) Proprietor: Viveros Villanueva Vides, S.L., 31251 Larraga (Navarra) (ES)
(72) Inventor: Álvarez Pérez, José Manuel, 31251 Larraga (Navarra) (ES); González García, Sandra, 31251 Larraga (Navarra) (ES); Cobos Román, Rebeca, 31251 Larraga (Navarra) (ES); Olego Morán, Miguel Ángel, 31251 Larraga (Navarra) (ES); Garzón Jimeno, José Enrique, 31251 Larraga (Navarra) (ES); Rubio Coque, Juan José, 31251 Larraga (Navarra) (ES)
(74) Representative: Toro Gordillo, Ignacio Maria

(56) References cited:
- EP-A1- 2 692 232
- SOUAD LOQMAN ET AL: "Antagonistic actinomycetes from Moroccan soil to control the grapevine gray mold", WORLD JOURNAL OF MICROBIOLOGY AND BIOTECHNOLOGY, KLUWER ACADEMIC PUBLISHERS, DO, vol. 25, no. 1, 4 October 2008 (2008-10-04), pages 81-91, XP019650434, ISSN: 1573-0972
- Wazer A. Hassam: "DEVELOPMENT AND SUPPRESSION OF GRAPEVINE BLACK FOOT CAUSED BY ILYONECTRIA RADICICOLA | Hassan | International Journal of Phytopathology", eSCI Journal of Plant pathology, 1 January 2012 (2012-01-01), pages 25-31, XP55272702, Retrieved from the Internet: URL:http://escijournals.net/index.php/phyt opath/article/view/43/110 [retrieved on 2016-05-13]
- Ricardo Feliciano ET AL: "ATIVIDADE ANTIFÚNGICA DE PRODUTOS ALTERNATIVOS SOBRE Ilyonectria liriodendri E Phaeomoniella chlamydospora", , 1 December 2013 (2013-12-01), XP55272705, Retrieved from the Internet: URL:http://www.conhecer.org.br/enciclop/20 13b/CIENCIAS AGRARIAS/atividade antifungica de.pdf [retrieved on 2016-05-13]

## Description

### OBJECT OF THE INVENTION

The present invention relates to the selection of actinobacteria of the grapevine rhizosphere (*Vitis vinifera*) or endophytic actinobacteria isolated from the grapevine plant root interior to reduce the infection rate of fungi that cause grapevine wood diseases by way of the root system by means of the application thereof on grapevine grafts prior to the establishment thereof in the nursery.

The object of the invention is to provide an economic technology, which is easy to use, and which can be applied during the industrial production process of grapevine plants in the nursery or which can even be applied to adult vineyards already established in the field in order to avoid or reduce the risk of infection by way of the root system by phytopathogenic fungi that cause grapevine wood diseases.

The method of the invention is especially applicable to grapevine plant nurseries with the aim of producing plants with an improved state of health or in companies dedicated to the grape production both table grapes and dedicated to the preparation of wine.

According to the method, a reduction in the levels of infection by the phytopathogenic fungi *Ilyonectria sp., Phaeomoniella chlamydospora* and *Phaeoacremonium aleophilum* of treated plants has been proven which varies between 73.1 and 76.9%, when it is compared with the level of infection detected in control grapevine grafts without treatment with actinobacteria.

A reduction of the mortality of grapevine grafts has also been observed in the establishment phase of soil nurseries for the production of grapevine plants which varies between 5.4% and 12.4% compared to the mortality rate observed in control plant batches without treatment.

### BACKGROUND OF THE INVENTION

Since the 1990s, the disease symptoms of grapevine wood in young vineyards (decaying of the grapevine) as well as the failures in the planting material have accounted for significant economic losses at a global level for the winegrowing sector. At present, it is considered that the fungi that cause grapevine wood diseases such as blackleg, Petri disease or the syndromes produced by species of the Botryosphaeriaceae family are one of the factors which most significantly contribute to the decaying of young grapevine plants [Gramaje and Armengol, 2011, Plant Dis. 95, 1040-1055]. Symptoms observed that stand out include low yields in berry production, stunted vine shoots and deficient development and weak development of the root system with the emergence of necrotic lesions both in the root and in the shoots. These symptoms lead to a pronounced delay in the growth of the plant and a high frequency of premature death.

The principal pathologies associated with the decaying of young grapevine plants are blackleg, produced by different species of the genus *Ilyonectria* (formerly *Cylindrocarpon*) [Hallen et. Al., 2006, Phytopathol. Mediterr. 45, S55-S67] and Petri disease, fundamentally produced by the *Phaeomoniella chlamydospora* fungi and different species of the genus *Phaeoacremonium* [Mundy and Manning, 2010, New Zealand Plant Protection 63: 160-166].

Different studies have demonstrated that many of the plants affected had been infected in the nursery during the different industrial production stages of grapevine plants. In fact, various times or stages of the process have been identified at which the contamination of the material used for the production of grapevine plants may be caused, fundamentally 1).- infection of the parent plants of American varieties from which the rootstocks are produced; 2).- infections produced during the grafting and formation stages of the callus between the rootstocks and the variety; 3).- infections produced during the establishment phase of the soil nursery [Gramaje and Armengol, 2011, Plant Dis. 95, 1040-1055].

The infection during the propagation process in the nursery is a significant problem since the pathogens are thereby introduced into the plant production cycle. The role of the grapevine nurseries as the origin of the contamination of a high number of plants has been demonstrated by Aroca et al. [Aroca et al., 2006, Eur. J. Plant Pathol. 115: 195-202] which in tracking carried out in grafted plants and American varieties of rootstocks they proved that 45.2% of the plants were infected with some fungus associated with the decaying of the grapevine, especially the species responsible for Petri disease.

In another study [Giménez-Jaime et al., 2006, J. Phytopathol. 154: 598-602] carried out in 6 nurseries in the Autonomous Community of Valencia, they proved the presence of fungi that cause wood diseases in all the nurseries analyzed. Specifically, *Cylindrocarpon spp., Botryospheria spp.* and *B*. *obtusa (D. seriata)* were preferably isolated in plant material corresponding to early phases of the production process (American variety of rootstocks and area of the graft in plants grafted prior to being planted in soil) in percentages which varied from 0.48% to 2.38% of the plants analyzed. On the contrary, *P. chlamydospora* and *P. aleophilum* were not detected until the cuttings were planted in the field to develop the roots and buds (aerial part) with infection percentages varying between 0 to 7.62% of the plants analyzed.

The problem is not exclusive to nurseries in Spain. In fact, *Cylindrocarpon spp.* has been isolated in South Africa from parent plants of the rootstocks and in this country and California this genus is considered the most significant pathogen in nurseries [Hallen et al., 2003, Australas. Plant Pathol. 32: 47-52.; Fourie and Hallen, 2004, Australasian Plant Pathol. 33: 313-315; Dubrovsky and Fabritius, 2007, Phytopathol. Mediterr. 46: 46-86].

The most significant phytopathogenic fungi indicated as responsible for the decaying of young grapevine plants have in common the capacity thereof to infect the plants by way of the root system. Thus, the presence of the fungi responsible for the blackleg disease has been demonstrated in the soil [Agustí-Brisach et al., 2014, Plant Pathol., 63: 316-322]. The fungi can remain here for long periods of time (in the form of chlamydospore or in saprophytic form) and penetrate the plant by way of the roots from the rooting phase in the nursery. Appropriate management of the soil can significantly reduce the risk of infection by way of the root. The capacity of *P*. *chlamydospora* and *P. aleophilum* for using this route of entry to infect grafts has also been demonstrated. In fact, Giménez-Jaime et al [Giménez-Jaime et al., 2006, J. Phytopathol. 154: 598-602] have described that these two species could only be isolated from grafts two months after being planted in the nursery. All these studies confirm that these pathogenic fungi can infect the grafts by way of the root system that is developed in the nursery.

In recent years, numerous studies have been developed aimed at reducing the risk of infection of grapevine plants in the nursery by means of using different strategies such as the use of different chemical fungicides, treatments of plant material with hot water, biocontrol or the development of varieties of rootstocks more resistant to the infection [for a review see Gramaje and Armengol, 2011, Plant Dis. 95, 1040-1055].

Biocontrol is one of the most promising technologies for controlling many pathologies without having to resort to the use of chemically synthesized pesticides with high toxicity in many cases and which have been frequently shown to be harmful to the environment and/or living beings. In grapevine nurseries, different tests with biocontrol agents (BCA) have been carried out. Fourie et al thus showed that the application of different strains of the fungus, *Trichoderma* had a positive effect for preventing infection by *Cylindrocarpon spp., P. chlamydospora* and *Phaeoacremonium spp.* In fact, lower levels of these fungi were observed in the roots of plants treated in the nursery [Fourie et al., 2001, Phytopathol. Mediterr. 40S: 473-478]. BCA *Trichoderma harzianum* has also been tested in grapevine nurseries for controlling infection by *P*. *chlamydospora.* BCA was applied in different ways: by means of immersion of the root mass in a suspension of spores, by means of immersion of the stems which are grafted on the plant foot so that they form the callus in a suspension of spores or by means of a combination of both. BCA showed certain efficacy by promoting the development of the root system and increasing the percentage of plants which survived the infection with the pathogen. However, an increase in the mortality of the plants treated with BCA was also observed as an adverse effect [De Marco and Osti, 2007, Phytopathol. Mediterr. 46: 73-83], therefore the efficacy of the application of BCA of the genus *Trichoderma* remains in question.

*Several other different attempts have been also carried out to control grapevine trunk diseases and also other pathologies by application of different kind of technologies. Thus, Dos Santos and colleagues [*Dos Santos et al., 2013, Enciclopedia Biosfera, 9(17): 3765-3774*)] have carried out different trials to control the fungi responsible of Petri disease and Black foot disease by using (alone or in combination) different chemical fungicides in vitro, although the use of biocontrol agents (bacterial strains) is not reported in this study. In another report one particular pathogen, Ilyonectria radicicola, involved in grapevine black foot is partially controlled by the application of a Trichoderma harzianum fungal strain as biocontrol agent [*Hassan et al., 2013, Int. J. Phytopathol. 2: 25-31*]. In recent years actinomycete strains have been tested as putative biocontrol agents to fight different fungal pathologies. In this way, actinomycete strains isolated from the rhizosphere of Vitis vinifera from Maroccan soils, mainly belonging to the Streptomyces genus, have been proved to effectively control both in vitro and by pre-inoculation of plantlets against Botrytis cinerea (grapevine gray mold) [*Loqman et al., 2009, Worl J. Microbiol. Biotechol. 25: 81-91*]. No information is reported in this manuscript about a putative use for controlling fungal pathogens involved* in *grapevine trunk diseases. More recently, the effectivity of different natural antifungal compounds to control the infection of fungi causing grapevine trunk diseases by inhibiting their penetration through pruning wounds have been reported [*Cobos et al., 2015, Appl. Environ. Microbiol. 81: 6474-6483*], although no information about the putative use of actinomycete strains to control these diseases is made in this report.*

### DESCRIPTION OF THE INVENTION

The technology that is advocated is intended to resolve a specific point of the previously stated problem, specifically reduce or limit as far as possible the infection of grapevine grafts in the nursery by way of the root system during the establishment phase of the graft nursery in soil, a time at which they develop the root system. To this end, we use 3 strains selected from actinobacteria as biocontrol agents (BCAs) when they are applied to grapevine grafts.

### A).- Strains Streptomyces sp. VV/E1, VV/R1 and VV/R4: description.

In one aspect, the invention is related to 3 strains of actinobacteria belonging to the genus *Streptomyces sp.* selected from the rhizosphere (strains VV/R1 and VV/R4) or from the root interior (strain W/E1) of grapevine plants that are 1 year old and which show high effectiveness for inhibiting growth, both in *in vitro* tests and in plant material, of phytopathogenic fungi *Ilyonectria sp., P. chlamydospora* and *P. aleophilum,* principally responsible for the decaying of young grapevine plants and various grapevine wood diseases.

The respective cultures of the strains of *Streptomyces sp.* called VV/E1, VV/R1 and VV/R4 are preserved and permanently deposited in the ISP5, ISP6, MBA, MEY, TBO and 2xTY, showing less growth in medium ISP4. This strain has a low sporulation capacity in medium MEY, apparently no sporing in mediums ISP1, ISP2, ISP3, ISP4, ISP5, ISP6, MBA, TBO and 2xTY.

In plates of medium ISP2, it is morphologically characterized by exhibiting rosacea coloring with rough morphology, a certain concentricity in the growth thereof with a swelling in the center.

### II).- Strain VV/R4.- The sequencing of ribosomal RNA (rRNA) 16S concluded that it is a Streptomyces strain which belongs to the taxonomic group S. capillispiralis-S. cellulosae-S. gancidicus-S. pseudogriseolus-S. werraensis, being indistinguishable based on the sequence of rRNA 16S since it shows a homology of 100%.

The strain VV/R1 has good growth in the mediums ISP1, ISP2, ISP3, ISP4, ISP5, MBA, MEY, TBO and 2xTY, showing less growth in medium ISP6. This strain has a very low sporulation capacity in medium ISP4, low sporulation capacity in medium ISP1, ISP7 and MBA; average sporulation capacity in ISP3 and MEY; high sporulation capacity in medium ISP2 and TBO. It does not shown sporulation capacity in medium ISP5 and 2xTY.

In plates of medium ISP2, it is morphologically characterized by producing colonies with a dark beige color with a flat morphology and concentric growth. They tend to spore in this medium (white color).

### B).- Isolation of the strains Streptomyces sp. W/R1, VV/R4 and VV/E1.

The mentioned strains have been isolated and selected from a total.of 85 analyzed strains of rhizospheric actinobacteria and 66 analyzed endophytic strains. Said strains were isolated from root rhizosphere samples in starch and casein medium or from the grapevine root interior according to the methodology described by Li et al [Li et al., 2012, Ant. Van Leeuwenhoek. 101:515-527]. The rhizosphere soil samples or root material were successively diluted in sterile saline solution (NaCl 0.9%). The dilutions were plated in a medium with starch and casein containing cycloheximide and nalidixic acid to selectively inhibit the growth of fungi and Gram(-) bacteria respectively. The plates were incubated for 7 days at 28 °C. All the colonies with a typical actinobacteria morphology (mycelial development, velvety or waxen aspect, possible production of aerial mycelium with or without exospores and possibility of producing diffusible pigments in the culture medium) were then replicated to a yeast-malt extract (micromineral enriched yeast, MEY) [Hopwood et al., 1985, Genetic manipulation of Streptomyces - A laboratory manual. The John Innes Foundation, Norwich, UK and Cold Spring Harbour Laboratory]. The provisional confirmation of it belonging to the group of actinobacteria took place by determining the filamentous morphology thereof following observation using an optical microscope.

### C).- Selection of the strains Streptomyces sp. VV/R1, VV/R4 and VV/E1.

The strains W/R1, VV/R4 and VV/E1 can be used to obtain grapevine plants grafted in grapevine nurseries with a much lower average level of infection by fungi that cause wood diseases (particularly *Ilyonectria sp., P. chlamydospora* and *P. aleophilum*). This application would allow the applicant company to have a plant material with a greatly improved state of health.

These strains can be used individually or together, in different combinations, for the application thereof in grapevine grafts by means of inclusion in the rooting hormone solution or for application on young and adult plants in the field by means of the application thereof to the root system by fertigation or other irrigation or winegrowing techniques with the aim of controlling the infection of grapevine plants by these phytopathogenic fungi by way of the root system.

The use of the strains W/R1, VV/R4 and VV/E1 in obtaining grafted grapevine plants provides numerous advantages. In particular, the use thereof in grapevine nurseries allows plants with an improved state of health to be obtained as the level of infection by fungi that cause wood diseases is reduced. These plants, due to the improved state of health thereof, are less prone to suffering subsequent infections once they are finally planted in the field, especially if periodic provisions of these microorganisms are made to the root system by means of fertigation or by any other alternative technique. The implementation of these microorganisms in the rhizosphere of plants or in the interior of the root system prevents or limits the infection by any fungus susceptible to antifungicidal mechanisms or compounds produced thereby.

### DESCRIPTION OF THE DRAWINGS

In order to complement the description below and with the aim of aiding a better understanding of the characteristics of the invention, according to a preferred practical exemplary embodiment of the same, a set of drawings is included as an integral part of said description, in which the following is depicted in an illustrative and non-limiting manner:

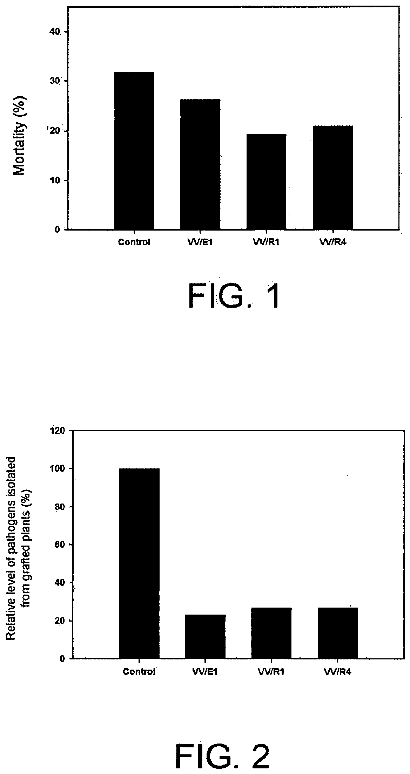

Figure 1.- shows a graph corresponding to the decrease in the average mortality rate observed in grapevines grafts established in a field nursery after the application of the selected actinobacteria W/E1, VV/R1 and VV/R4 in comparison with the average mortality rate observed in untreated control plants.
Figure 2.- shows a graph corresponding to the relative decrease (%) of the pathogenic fungi *Ilyonectria sp., P. chlamydospora* and *P*. *aleophilum* observed in grapevine grafts established in a field nursery after the application of the selected actinobacteria W/E1, VV/R1 and VV/R4 in comparison with the average infection rate observed in untreated control plants.

### PREFERRED EXEMPLARY EMBODIMENTS OF THE INVENTION

The following examples illustrate the invention, although they should not be considered as limiting to the scope of the same.

### *Example 1. Obtaining and charactering the strains VV/R1, VV/R4 and VV/E1.

A total of 85 strains of rhizospheric actinobacteria and 66 endophytic strains isolated from 1 year old grapevine plants were distinguished as a function of the easily observable morphological features (color and morphology of the colony, capacity to form aerial mycelium, capacity to spore, production of soluble and diffusible pigments in the culture medium). All these strains were tested for the capacity thereof to inhibit the growth of the phytopathogenic fungi *Ilyonectria sp.* and *Diplodia seriata* due to the aggressive growth thereof. To this end, the biotest technique was used. All the strains were grown at 28 °C in plates of malt agar extract (MEA) for 5 days. A block of agar was then placed in the center of the plate, containing mycelium of the previously mentioned phytopathogenic fungi at a distance of 2 cm from the edge of the colony of tested actinobacteria. The plates were then incubated at 25 °C for 5 to 10 days in order to determine the possible inhibitory effect of the actinobacteria strain against the phytopathogenic fungi. A total of 1 endophytic actinobacteria (W/E1) and 2 rhizospheric strains (VV/R1 and VV/R4) were selected due to the capacity thereof to effectively inhibit *in vitro* the growth of both pathogens. The capacity to inhibit the growth was determined by means of the methodology of Lamsal et al (2012) [Mycobiology 40: 244-251], calculating the inhibition rate (IR, %) of each one of the actinobacteria using the following expression: IR (%) = [1-(Da-D)/(Dc-D)]x100; where Da is the growth of the fungus in the antagonism test, Dc is the diameter of growth in the control (no antagonism with any actinobacteria) and D is the diameter of the block of agar which contained the fungus (7 mm). This experiment was carried out three times for each one of the actinobacteria. In this test, the strain VV/E1 showed an inhibition of growth against *D. seriata* of 56.8 (± 6.1)% and 38.8 (± 2.9)% against *l*. *macrodidyma.* The strain VV/R1 respectively showed an inhibition of growth of 48.8 (± 3.9)% and 53.7 (± 4.2)% against *D. seriata* and *l. macrodidyma* respectively. The strain VV/R4 was capable of inhibiting *in vitro* the growth of *D. seriata* by a percentage of 51.7% (± 6.2) and the growth of *l. macrodidyma* by 50.8% (± 3.7).

The 3 preselected strains also showed a high capacity for inhibiting *in vitro* the growth of other phytopathogenic fungi such as *Cadophora luteo-olivacea, Eutypa lata, Ilyonectria sp., Phaeomoniella chlamydospora* and *Phaeoacremonium aleophilum.*

### *Example 2. Production of biomass of the strains VV/R1, VV/R4 and VV/E1.

Two different methodologies were used with the aim of testing the versatility of each strain at the time of producing the biomass.

### 2.1).- Production of biomass due to growth in flasks under aerobic conditions.

To this end, 1 I flasks which contained 0.2 I of malt extract broth medium were used. Each flask was inoculated with 5 ml of a culture developed in the same medium of D.O. 1 spectrophotometrically estimated at 600 nm. The flasks were incubated at 28 °C with constant agitation of 220 rpm in an orbital incubator. The growth was determined each 24 hours. During the process, the homogeneity of the culture was tested by means of direct observation using an optical microscope. Once the incubation was finalized, the cellular viability was checked by means of counting colony-forming units per ml (UFC/ml). All the strains grew very quickly in these conditions, obtaining a large quantity of biomass and viability equal to or greater than that of the *S*. *coelicolor* species used as a control.

### 2.2).- Production of biomass in fermenter.

The study was carried out in a 2.5 l fermenter of the laboratory, Biostat-A, controlling the different parameters that affect the strains such as dissolved O2, pH, foam, acidity and temperature. In order to develop the cultures, 1.5 I of malt extract broth medium was used which was inoculated with a preculture of each bacteria until a cellular suspension of D.O. = 0.1 (determined at 600 nm) was obtained. The pH of the culture was maintained constant at a value of 7.0 and the incubation temperature was 28 °C. Lastly, the concertation of dissolved oxygen was maintained constant at 35%. The growth and cellular viability were monitored as indicated in the previous section. Under these conditions, all the strains showed quick and homogenous biomass production with high cellular viability, of the same order of magnitude as the strain *S*. *coelicolor* used as a control.

### *Example 3. Tests in the field: application of the strains Streptomyces sp. VV/R1, VV/R4 and VV/E1 to grapevine grafts.

The 3 preselected strains of actinobacteria - endophytic actinobacteria (W/E1) and 2 rhizospheric strains (VV/R1 and VV/R4) - were grown in a malt extract broth medium by means of inoculation of 200 ml of medium in 1 l flasks at 28 °C and 220 rpm of agitation for 120 hours.

The microbial biomass was precipitated by centrifugation at 12,000 xg for 20 minutes at 4 °C. It was then re-suspended in 50 ml of glycerol of 20% (m/v) and was preserved at 20 °C until the application thereof.

Immediately prior to the application thereof, the number of bacteria present in each preparation was quantified by sowing successive dilutions in PDA medium and incubating at 28 °C for 5 days.

The application of the bacteria to the grafts was carried out via batches of 100 grafts (rootstocks R-110 and white tempranillo variety) by immersing the basal portion of the rootstocks in 2 I of a solution containing root hormone to which is added 10⁷ bacterial cells per ml in the form of a suspension in glycerol of 20% (m/v). In parallel, a negative control of grafts was carried out to which was added an equivalent quantity of glycerol of 20% without bacteria.

The grafts are maintained in a root solution with bacteria for 24 hours. After this time, they are planted in a field for the establishment of an experimental nursery which is maintained in optimal conditions by specialist personnel from Viveros Villanueva Vides S.L. by means of the provision of the normal fertigation program which the company uses.

Seventy days after the establishment thereof in the soil, a check of the grafts was carried out consisting of: determining the mortality rate (grafts not sprouted against total grafts placed in the field), total height of the plant and length of seventh internode.

The plants were pulled out 200 days after the establishment thereof, in the field and the level of infection by the pathogenic fungi *Ilyonectria sp, P. chlamydospora* and *P. aleophilum* was determined at the root insertion point with the aim of determining the possible protective effect of the actinobacteria applied to prevent the infection of these fungi by way of the root system. The analysis was carried out according to the methodology described by Giménez-Jaime et al [Giménez-Jaime et al., 2006, J. Phytopathol. 154: 598-602] and Aroca et al [Aroca et al., 2006, Eur. J. Plant Pathol. 115: 195-202].

The identification of the isolated fungi was carried out as indicated below.
- In order to identify the species of the Botryosphaeriaceae family, the classic morphological criteria were followed [Phillips et al., 2013, Studies in Mycology 76:51-167] and the molecular techniques described for this group [Alves et al., 2007, Res. Microbiol. 158:112-21; Martos et al., 2009, Phytopathol. Mediterr. 48:162; Spagnolo et al. 2011, Europ. J. Plant Pathol. 129: 485-500].
- The different species of the genus *Phaeoacremonium* were identified following the electronic identification code developed by Mostert et al [Mostert et al. 2006, Studies in Mycology 54:1-113]. Specific oligonucleotides were also used to detect *P. aleophilum* [Tegli et al., 2000, Phytopathol. Mediterr. 39:134-49].
- The identification of the *Ilyonectria* species was carried out taking into account the recent reclassification of some species of the genus "Cylindrocarpon" [Cabral et al., 2012, Fungal biology 116:62-80.] associated with wood diseases.
- The identification of *Phaeomoniella chlamydospora* was developed according to the classic morphological criteria [Crous and Gams, 2006, Phytopathol. Mediterr. 39: 112-18] and the specific detection by PCR [Tegli et al., 2000, Phytopathol. Mediterr. 39:134-49].

The tests in the field were carried out over 2 consecutive years in 3 batches of 25 plants per treatment and year.

The tests in the field revealed that:
A).- The control grafts established in the field nursery showed an average mortality rate of 31.7%. The application of the actinobacteria to the grafts produced a decrease in the mortality to 26.3% -strain VV/E1- (decrease in mortality with respect to the control of 17.0%); a decrease in the mortality to 19.3% -strain VV/R1- (decrease in mortality with respect to the control of 39.1%) and a decrease in the mortality to 21.0% -strain VV/R4- (decrease in mortality with respect to the control of 33.8%), as can be observed in **Figure 2****.**
B).- The infection rate of the grafts established in the field nursery reduced significantly after the application of the selected actinobacteria (**Figure 3**). In fact, assigning a relative value of 100 for the level of infection by the pathogenic fungi *Ilyonectria sp., P. chlamydospora* and *P. aleophilum* in grafted control plants (untreated with actinobacteria) and established in the field nursery, when the growing seasons ended, it can be observed how the level of infection reduced to 26.9% (decrease of 73.1%) in the case of plants treated with the strains VV/R1 and VV/R4, while the level of infection decreased to 23.1% (decrease of 76.9%) in the case of plants treated with the actinobacteria VV/E1.

## Claims

1. A product *suitable for* controlling phytopathogenic fungi that cause grapevine wood diseases, **characterized *in that*** *it has* a *composition comprising* the strain of the genus *Streptomyces sp.,* deposited in the CECT with the access number CECT 8852 (strain *Streptomyces sp.* W/E1).

2. A product *suitable for* controlling phytopathogenic fungi that cause grapevine wood diseases, **characterized *in that*** *it has* a *composition comprising* the strain of the genus *Streptomyces sp.,* deposited in the CECT with the access number CECT 8853 (strain *Streptomyces sp.* W/R1).

3. A product *suitable for* controlling phytopathogenic fungi that cause grapevine wood diseases, **characterized *in that*** *it has* a *composition comprising* the strain of the genus *Streptomyces sp.,* deposited in the CECT with the access number CECT 8854 (strain *Streptomyces sp.* VV/R4).

4. A method *for* controlling phytopathogenic fungi that cause grapevine wood diseases according to the products of claims 1 to 3, **characterized in that** it consists of the application, individually, or in any combination of said products, to grapevine grafts during the treatment phase with root hormone (or any other phase prior to the establishment of the graft nursery in soil) to obtain grapevine plants with a lower rate of infection by fungi that cause grapevine wood diseases, *and*/*or to achieve a reduction in the mortality rate of grafts established in the nursery.*

5. *A method for controlling phytopathogenic fungi that cause grapevine wood diseases according to the products and method of claims 1 to 4, **characterized in that** it consists of the application, individually, or in any combination of said products, to the root system of young and adult plants in the field by means of fertigation or any other alternative technique for controlling fungi that cause grapevine wood diseases.*

## Patentansprüche

1. Produkt, *geeignet zur* Bekämpfung von phytopathogenen Pilzen, die Weinrebenholzkrankheiten verursachen, **dadurch gekennzeichnet, *dass*** *es eine Zusammensetzung umfasst, die* den Stamm der Gattung *Streptomyces sp.* umfasst, der im CECT mit der Zugangsnummer CECT 8852 hinterlegt ist (Stamm *Streptomyces sp.* W/E1).

2. Produkt, *geeignet zur* Bekämpfung von phytopathogenen Pilzen, die Weinrebenholzkrankheiten verursachen, **dadurch gekennzeichnet, *dass*** *es eine Zusammensetzung umfasst, die* den Stamm der Gattung *Streptomyces sp.* umfasst, der im CECT mit der Zugangsnummer CECT 8853 hinterlegt ist (Stamm *Streptomyces sp.* W/R1).

3. Produkt, *geeignet zur* Bekämpfung von phytopathogenen Pilzen, die Weinrebenholzkrankheiten verursachen, **dadurch gekennzeichnet, *dass*** *es eine Zusammensetzung umfasst, die* den Stamm der Gattung *Streptomyces sp.* umfasst, der im CECT mit der Zugangsnummer CECT 8854 hinterlegt ist (Stamm *Streptomyces sp.* W/R4).

4. Verfahren *zur* Bekämpfung von phytopathogenen Pilzen, die Weinrebenholzkrankheiten verursachen, gemäß den Produkten nach Anspruch 1 bis 3, **dadurch gekennzeichnet, dass** es aus der Anwendung, einzeln oder in beliebiger Kombination dieser Produkte, auf Weinreben-Pfropfreiser während der Behandlungsphase mit Wurzelhormon (oder einer beliebigen anderen Phase vor der Erzeugung des Bestands für Pfropfreiser im Boden) besteht, um Weinrebenpflanzen mit einer geringeren Infektionsrate durch Pilze zu erhalten, die Weinrebenholzkrankheiten verursachen, *und*/*oder um eine Verringerung der Mortalitätsrate von im Bestand erzeugten Pfropfreisern zu erzielen.*

5. *Verfahren zur Bekämpfung von phytopathogenen Pilzen, die Weinrebenholzkrankheiten verursachen, gemäß den Produkten und dem Verfahren nach Anspruch 1 bis 4, **dadurch gekennzeichnet, dass** es aus der Anwendung, einzeln oder in beliebiger Kombination dieser Produkte, auf das Wurzelsystem von jungen und ausgewachsenen Pflanzen auf dem Feld mittels Fertigation oder einer anderen alternativen Technik zur Bekämpfung von Pilzen besteht, die Weinrebenholzkrankheiten verursachen.*

## Revendications

1. Produit *adapté pour* le contrôle des champignons phytopathogènes qui causent des maladies du bois de vigne, **caractérisé *en ce qu'****il a une composition comprenant* la souche du genre *Streptomyces* sp., déposée au CECT avec le numéro d'accès CECT 8852 (souche *Streptomyces sp.* W/E1).

2. Produit *adapté pour* le contrôle des champignons phytopathogènes qui causent des maladies du bois de vigne, **caractérisé *en ce qu'****il a une composition comprenant* la souche du genre *Streptomyces* sp., déposée au CECT avec le numéro d'accès CECT 8853 (souche *Streptomyces sp.* W/R1).

3. Produit *adapté pour* le contrôle des champignons phytopathogènes qui causent des maladies du bois de vigne, **caractérisé *en ce qu'****il a une composition comprenant* la souche du genre *Streptomyces* sp., déposée au CECT avec le numéro d'accès CECT 8854 (souche *Streptomyces sp.* W/R4).

4. Procédé *pour* le contrôle contre les champignons phytopathogènes qui causent des maladies du bois de vigne selon les produits des revendications 1 à 3, **caractérisé en ce qu'**il consiste en l'application, individuellement ou toute combinaison desdits produits, aux greffons de vigne pendant la phase de traitement avec l'hormone racinaire (ou toute autre phase avant l'implantation de la pépinière de greffons dans le sol) pour obtenir des plants de vigne avec un plus faible taux d'infection par des champignons qui causent des maladies du bois de vigne, *et*/*ou pour obtenir une réduction du taux de mortalité des greffons implantés en pépinière.*

5. *Procédé pour le contrôle des champignons phytopathogènes qui causent des maladies du bois de vigne selon les produits et procédé des revendications 1 à 4, **caractérisé en ce qu'**il consiste en l'application, individuellement ou toute combinaison desdits produits,* au *système racinaire de plants jeunes et adultes dans le domaine moyennant la fertirrigation ou toute autre technique alternative pour contrôler les champignons qui causent les maladies du bois de vigne.*
